(19) Europäisches Patentamt / European Patent Office / Office européen des brevets

(11) **EP 3 733 873 A1**

(12) **EUROPEAN PATENT APPLICATION**
published in accordance with Art. 153(4) EPC

(43) Date of publication:
**04.11.2020 Bulletin 2020/45**

(21) Application number: **18893766.8**

(22) Date of filing: **18.10.2018**

(51) Int Cl.:
*C12Q 1/6851* (2018.01)     *C40B 50/06* (2006.01)
*G01N 33/02* (2006.01)     *G01N 33/15* (2006.01)
*G01N 33/50* (2006.01)

(86) International application number:
**PCT/JP2018/038789**

(87) International publication number:
**WO 2019/130745 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2017 JP 2017252095**

(71) Applicants:
• **Suntory Holdings Limited**
**Osaka 530-8203 (JP)**
• **Mie University**
**Mie 514-8507 (JP)**

(72) Inventors:
• **YAMANAKA, Mikiko**
**Soraku-gun, Kyoto 619-0284 (JP)**
• **TANAKA, Toshio**
**Tsu-shi, Mie 514-5807 (JP)**
• **KOIWA, Junko**
**Tsu-shi, Mie 514-5807 (JP)**

(74) Representative: **Vossius & Partner**
**Patentanwälte Rechtsanwälte mbB**
**Siebertstrasse 3**
**81675 München (DE)**

(54) **METHOD FOR PRODUCING LIBRARY FOR USE IN PREDICTION OF ACTION OF SUBSTANCE OF INTEREST ON LIVING BODY, AND METHOD FOR EVALUATING ACTION OF SUBSTANCE OF INTEREST ON LIVING BODY BASED ON CHANGE IN ACTION PARAMETER OR THE LIKE OF ZEBRAFISH**

(57)     A method for producing a library for use in prediction of the action of a substance of interest on the living organism, the library having a large number of numerical data obtained based on change in a behavioral parameter of zebrafish and a large number of numerical data representing change in expression levels of genes integrated and recorded therein, the method including the steps of: preparing a substance of known action that is a substance whose action on the living organism is known; measuring a behavioral parameter of zebrafish not given the substance of known action in a bath having the zebrafish therein; administering the substance of known action to the bath having the zebrafish therein and measuring the behavioral parameter of the zebrafish after the administration of the substance of known action in the bath having the zebrafish therein; detecting change in the behavioral parameter of the zebrafish by the administration of the substance of known action; subdividing data representing the change in the obtained behavioral parameter and converting the data into a large number of numerical data; normalizing the large number of numerical data obtained based on the change in the behavioral parameter; normalizing a large number of numerical data representing the change in expression levels of genes after the administration of the substance of known action; and integrating and recording the large number of numerical data obtained based on the change in the behavioral parameter and the large number of numerical data representing change in expression levels of genes, both normalized.

**Description**

TECHNICAL FIELD

The present invention relates to a method for producing a library for use in prediction of the action of a substance of interest on the living organism and a method for evaluating the action of a substance of interest on the living organism based on the change in a behavioral parameter of zebrafish.

BACKGROUND ART

There are evaluation systems mainly in rodents in order to search for materials, particularly food materials, that can exhibit action effective for humans.

Known methods for evaluating the central nervous system effect using rodents include methods such as elevated plus maze, tail suspension, forced swimming, or the like. For example, forced swimming tests using rats are used as methods for screening antidepressants (see Non Patent Literature 1).

CITATION LIST

- Non Patent Literature

Non Patent Literature 1: Porsolt et al., Nature, 266, 730-732, 1977
Non Patent Literature 2: Johnson A, Hamilton TJ. (2017) Modafinil decreases anxiety-like behaviour in zebrafish. PeerJ 5: e2994

SUMMARY OF INVENTION

- Technical Problem

By any one method for evaluation of such methods for evaluating the central nervous system effect using rodents as described in Non-Patent Literature 1, it is not possible to evaluate a plurality of central nervous system effects.

Moreover, the methods for evaluation are methods often used in tests for evaluating pharmaceutical preparations and often fail to provide clear evaluation, when the methods for evaluation are applied to foods, which, unlike pharmaceutical preparations, exhibit slow action on the living organism.

Moreover, the methods for evaluating the central nervous system effect using rodents have problems of cost and time required for the evaluation.

An evaluation system using a model organism that substitutes rodents is demanded, since the evaluation systems mainly in rodents have some problems, as seen above.

On this point, zebrafish attracts attention as a model organism that substitutes for rodents since the total genome sequence of zebrafish is about 80% homologous to the human genome and zebrafish has major organs equivalent to humans.

Non-Patent Literature 2 discloses that by giving modafinil to zebrafish and conducting the behavioral analysis of zebrafish in bath, it has been suggested that modafinil has antianxiety action.

In Non-Patent Literature 2, it is concluded that by comparing the result of the behavioral analysis of zebrafish where ethanol, which is known to have antianxiety action, is given to zebrafish and the tendency of the result of the behavioral analysis where modafinil is given to zebrafish, it was suggested that modafinil has antianxiety action.

Here, it is considered that, when the effect of a food or the like on the living organism is evaluated, particularly action such as central nervous system effect, the expression of genes involved in the action should be considered, but the link between the administration of modafinil and the change in expression levels of genes has not been considered in Non-Patent Literature 2.

Currently, to link the action such as the central nervous system effect on the living organism and the change in expression levels of genes, researchers often speculate particular genes associated with the action and examine, on the basis of the speculation, whether there is correspondence therebetween.

Linking in line with the researcher's speculation and subjectivity is possible in this case, but, if the expression of unexpected genes that are unexpectable for the researchers is associated with action, then it has been difficult to discover the link.

With such a background, a method for exhaustively linking, with the administration of a substance of interest, and evaluating the change in expression levels of genes and the action of the substance of interest on the living organism, including a range that is not included in the prediction by researchers has been desired.

The present invention is directed to provide a method for exhaustively linking, with the administration of a substance of interest, and evaluating the change in expression levels of genes and the action of the substance of interest on the living organism using zebrafish and to provide a method for producing a library for use in prediction of the action of a substance of interest on the living organism.

- Solution to Problem

The present inventors have found that the action of a substance of interest on the living organism can be predicted by linking the behavioral pattern of zebrafish upon the administration of the substance of interest and expression levels of genes, thereby completing the present invention.

The present invention includes the following methods.

[1] A method for producing a library for use in prediction of the action of a substance of interest on the living organism, the library having a large number of numerical data obtained based on change in a behavioral parameter and the like of zebrafish and a large number of numerical data representing change in expression levels of genes integrated and recorded therein, the method including the steps of:

preparing a substance of known action that is a substance whose action on the living organism is known;
measuring a behavioral parameter of zebrafish not given the substance of known action in a bath having the zebrafish therein;
administering the substance of known action to the bath having the zebrafish therein and measuring the behavioral parameter of the zebrafish after the administration of the substance of known action in the bath having the zebrafish therein;
detecting change in the behavioral parameter of the zebrafish by the administration of the substance of known action;
subdividing data representing the change in the obtained behavioral parameter and converting the data into a large number of numerical data;
normalizing the large number of numerical data obtained based on the change in the behavioral parameter;
normalizing a large number of numerical data representing the change in expression levels of genes after the administration of the substance of known action; and
integrating and recording the large number of numerical data obtained based on the change in the behavioral parameter and the large number of numerical data representing change in expression levels of genes, both normalized.

[2] The method for producing a library according to [1], wherein the substance of known action is an active ingredient of a pharmaceutical preparation.
[3] The method for producing a library according to [1] or [2], wherein
the substance of known action is a substance having an already known relation between the administration of the substance of known action and change in expression levels of genes, and
the step of normalizing a large number of numerical data representing the change in expression levels of genes after the administration of the substance of known action includes the steps of:

obtaining a large number of numerical data representing change in expression levels of genes after the administration of the substance of known action; and
normalizing the large number of numerical data, with reference to the relation already known.

[4] The method for producing a library according to [1] or [2], wherein
the substance of known action is a substance having an unknown relation between the administration of the substance of known action and change in expression levels of genes, and
the step of normalizing a large number of numerical data representing change in expression levels of genes after the administration of the substance of known action includes the steps of:

extracting mRNA from each of control zebrafish not given the substance of known action and zebrafish after the administration of the substance of known action, and measuring expression quantities of mRNAs to determine a sequence of mRNA whose expression quantity has been changed;
identifying a gene whose expression level has changed correlationally by the administration of the substance of known action based on the sequence of the mRNA whose expression quantity has changed in a comparison between the control zebrafish and the zebrafish after the administration of the substance of known action, and obtaining a large number of numerical data representing change in expression levels of genes after the administration of the substance of known action; and

normalizing the large number of numerical data.

[5] The method for producing a library according to any of [1] to [4], wherein the substance of known action is a substance having central nervous system effect.

[6] The method for producing a library according to any of [1] to [5], wherein the behavioral parameter is at least one behavioral parameter selected from the group consisting of the mobility state, the distance moved total, the in zone frequency, the distance to zone, and the turn angle of zebrafish.

[7] A method for evaluating the action of a substance of interest on the living organism, including the steps of:

preparing a library having a large number of numerical data obtained based on change in a behavioral parameter and the like of zebrafish and a large number of numerical data representing change in expression levels of genes, both normalized, integrated, and recorded therein;

measuring a behavioral parameter of zebrafish not given a substance of interest in a bath having the zebrafish therein;

administering the substance of interest to the bath having the zebrafish therein and measuring the behavioral parameter of the zebrafish after the administration of the substance of interest in the bath having the zebrafish therein;

normalizing a large number of numerical data obtained based on change in the behavioral parameter of zebrafish by the administration of the substance of interest;

identifying a gene whose expression level is changed in correlation with the administration of the substance of interest by statistically matching and analyzing the large number of numerical data obtained based on change in the behavioral parameter of zebrafish by the administration of the substance of interest and the large number of numerical data obtained based on change in the behavioral parameter of zebrafish and the large number of numerical data representing change in expression levels of genes integrated and recorded in the library, both normalized;

thereby predicting action of the substance of interest on the living organism based on function of the identified gene.

[8] The method for evaluation according to [7], wherein the behavioral parameter is at least one behavioral parameter selected from the group consisting of the mobility state, the distance moved total, the in zone frequency, the distance to zone, and the turn angle of zebrafish.

[9] The method for producing a library according to any of [1] to [6] or method for evaluation according to [7] or [8], wherein the substance of interest is a food or a component derived from a food.

[10] The method for evaluation according to any of [7] to [9], further including the steps of: extracting mRNA from each of control zebrafish not given the substance of interest and zebrafish after the administration of the substance of interest, and measuring expression quantities of mRNAs to determine a sequence of mRNA whose expression quantity has been changed; identifying a gene whose expression level has changed correlationally by the administration of the substance of interest based on the sequence of the mRNA whose expression quantity has changed in a comparison between the control zebrafish and the zebrafish after the administration of the substance of interest, and obtaining a large number of numerical data representing change in expression levels of genes after the administration of the substance of interest; and normalizing a large number of numerical data representing the change in expression levels of genes after the administration of the substance of interest; thereby obtaining the large number of numerical data representing the change in expression levels of genes after the administration of the substance of interest; and

estimating novel action or mechanism of the substance of interest by statistically matching and analyzing the large number of numerical data obtained based on change in the behavioral parameter of zebrafish by the administration of the substance of interest and the large number of numerical data representing the change in expression levels of genes after the administration of the substance of interest, both normalized, and the large number of numerical data obtained based on change in the behavioral parameter of zebrafish and a large number of numerical data representing change in expression level of a gene integrated and recorded in the library.

- Advantageous Effects of Invention

The present invention can provide a method for exhaustively linking, with the administration of a substance of interest, and evaluating the change in expression levels of genes and the action of the substance of interest on the living organism using zebrafish.

Moreover, a method for producing a library for use in prediction of the action of a substance of interest on the living organism can be provided.

BRIEF DESCRIPTION OF DRAWINGS

FIG. 1 illustrates the result of cluster analysis with the Euclid distance.
FIG. 2 illustrates the result of cluster analysis with the Manhattan distance.
FIG. 3 illustrates the result of cluster analysis with the maximum distance.
FIG. 4 illustrates the result of cluster analysis with the Euclid distance.
FIG. 5 is an enlarged view of the region surrounded with a dotted line in FIG. 4.

DESCRIPTION OF EMBODIMENTS

The method for evaluating the action of a substance of interest on the living organism according to the present invention (hereinafter, also referred to as, the method for evaluation according to the present invention) is a method including the steps of:

preparing a library having a large number of numerical data obtained based on change in a behavioral parameter and the like of zebrafish and a large number of numerical data representing change in expression levels of genes, both normalized, integrated, and recorded therein;
measuring a behavioral parameter of zebrafish not given a substance of interest in a bath having the zebrafish therein, administering the substance of interest to the bath having the zebrafish therein and measuring the behavioral parameter of the zebrafish after the administration of the substance of interest in the bath having the zebrafish therein, normalizing a large number of numerical data obtained based on change in the behavioral parameter of zebrafish by the administration of the substance of interest,
identifying a gene whose expression level is changed in correlation with the administration of the substance of interest by statistically matching and analyzing the large number of numerical data obtained based on change in the behavioral parameter of zebrafish by the administration of the substance of interest and the large number of numerical data obtained based on change in the behavioral parameter of zebrafish and the large number of numerical data representing change in expression levels of genes integrated and recorded in the library, both normalized,
thereby predicting action of the substance of interest on the living organism based on function of the identified gene.

As for Zebrafish (Danio rerio), it is known that the total genome sequence has 80% homology in comparison with the human genome, the number of genes thereof is also about the same as that of humans, and the development and the structure of major organs and tissues resemble closely to those of humans. Zebrafish can be preferably used in the method for evaluation according to the present invention since major organs are formed in 6 to 7 dpf (days post fertilization, 6 to 7 day-embryo).

Moreover, zebrafish is suitable in applying the method for evaluation according to the present invention to many substances of interest with different conditions at a time since approximately 200 or more fertilized eggs can be obtained in laying eggs at once and many Zebrafish individuals with the same genetic background can be obtained at the same time.

In the present invention, it is preferred to use at the same time zebrafish having the same genetic background born at the same time. This is because use of zebrafish having the same genetic background born at the same time can reduce the effect of the individual difference of zebrafish to the extent that can be ignored.

The behavioral parameter of zebrafish can be measured with an apparatus that records the behavior of zebrafish in a water bath. Examples of the apparatus for recording the behavior of zebrafish that can be used include Danio Vision (manufactured by Noldus Information Technology), and the like.

Such a recording apparatus is equipped with a platform for a well plate (48-well, 96-well and the like), a light, an infrared camera, and the like.

In the well plate, for example, $0.3 \times$ Danieau's solution (19.3 mM NaCl, 0.23 mM KCl, 0.23 mM $MgSO_4$, 0.2 mM $Ca(NO_3)_2$, 1.7 mM HEPES, pH 7.2) may be placed as a water bath. The water bath and an individual of zebrafish are placed in each well and the behavioral parameter is measured.

To measure the behavioral parameter and the like of zebrafish when a substance of interest is administered, the substance of interest is administered to each well at a predetermined concentration.

At the same time, the behavioral parameter of zebrafish in a well to which the substance of interest is not administered is measured as a control to be compared.

Since the kind and the concentration of the substance of interest can be changed for each well, it is possible to apply the method for evaluation according to the present invention to many substances of interest with different conditions at a time.

Examples of the behavioral parameter of zebrafish include the following indicators.

Mobility state
Distance moved total (DMF)
In zone frequency (IZF)
Distance to zone (DTZ)
Turn angle (TA)

The mobility state indicates the mobility state of zebrafish obtained with the software Ethovision as described below during the measurement period.

Herein, mobility states obtained with Ethovision are classified into 3 stages: high speed (mobility states equal to or higher than 65 and equal to or lower than 95), moderate speed (mobility states equal to or higher than 35 and lower than 65), and low speed (mobility states equal to or higher than 5 and lower than 35) to be used.

DMF is the distance moved total of zebrafish during the measurement period and represented by a high numerical value when the distance moved total is long.

IZF is the number of times zebrafish passes through a central circle (a radius of 2 mm) during the measurement period and represented by a high numerical value when the number of times of passing is high.

DTZ is an indicator about whether zebrafish has been in the vicinity of or has been away from the central circle during the measurement period and represented by a low numerical value when the duration being in the vicinity of the central circle is long.

TA is an indicator of frequency of zebrafish changing the direction of travel during the measurement period and represented by a high numerical value when the frequency of changing the direction of travel is high.

Any of these indicators can be measured by a software (Ethovision, manufactured by Noldus Information Technology) attached to the apparatus for recording the behavior of zebrafish.

In the method for evaluation according to the present invention, a library is used having the relations with behavioral parameters and the like and expression levels of genes of zebrafish recorded therein.

The library is prepared and saved on a recording medium before the working of the method for evaluation according to the present invention using a substance of interest.

In the library, a large number of numerical data obtained based on change in a behavioral parameter or the like of zebrafish and a large number of numerical data representing change in expression levels of genes, both normalized are integrated and recorded.

In the method for evaluation according to the present invention, by statistically matching and analyzing a large number of numerical data obtained based on change in the behavioral parameter of zebrafish by the administration of a substance of interest and a large number of numerical data obtained based on change in the behavioral parameter of zebrafish and a large number of numerical data representing change in expression levels of genes integrated and recorded in the library, both normalized, a gene whose expression level is changed in correlation with the administration of the substance of interest is identified.

The analysis can be performed by a bioinformatic technique after conducting pretreatment (such as normalization) of data.

For example, it can be performed by a technique such as the multivariate analysis or the network analysis.

Specific examples of the multivariate analysis include general cluster analyses.

Examples of the cluster analyses include hierarchical clustering (similarity: Euclid distance, Manhattan distance, or maximum distance)

Examples also include non-hierarchical clustering (K-means, self-organizing map).

Moreover, examples of the network analysis include analyses using the Bayesian network or the Boolean network.

These analyses can be performed with a personal computer equipped with a processing software.

A large number of numerical data that is subdivided data representing change in the mobility state, the distance moved total, the in zone frequency, the distance to zone, and the turn angle, illustrated as examples of the indicators of the behavioral parameters, is recorded in the library.

The subdivision of data representing the change in the behavioral parameters can be performed, for example, by the following methods.

- Making threshold intervals for digitizing an indicator small

- Altering the measurement duration of a behavioral parameter
- Extracting data only during a particular period from those measured for a behavioral parameter

Moreover, the data representing the change in the behavioral parameters and the like may be recorded as a large number of numerical data as further subdivided data in combination with data about stress given in zebrafish.

Examples of the stress include the repetition of light and dark periods, light period stimulation at a certain time, change in water temperature, stimulation with a chemical substance, infrared stimulation, ultraviolet stimulation, electrical stimulation, sound stimulation, vibration stimulation, and the like.

The subdivision of data representing the change in the behavioral parameters in combination with these data about stress can be performed, for example, by the following methods.

- Altering the durations of the light and dark periods when repeating light and dark periods
- Extracting data of particular light or dark periods when repeating light and dark periods
- Altering the ways of providing the stimulation when giving light period stimulation

When subdividing data that is combined data of data representing the change in the behavioral parameters and the like and the data about stress, the number of data representing the change in the behavioral parameters and the like is multiplied by the number of the ways of giving stress.

Therefore, a large number of numerical data obtained based on the change in the behavioral parameters and the like, subdivided according to the conditions of providing stress and conditions of extracting data are recorded in the library.

The large number of numerical data obtained based on the change in the behavioral parameters and the like, described above are normalized and then integrated with a large number of numerical data representing change in expression levels of genes and recorded in the same library.

As for the large number of numerical data to be recorded in the library, it is preferred that the number of data representing change in expression levels of genes and the number of data representing the change in the behavioral parameters and the like are equivalent.

The library can be produced and prepared by the following procedure.

Such a method for producing a library is a method for producing a library according to the present invention.

The method includes:

preparing a substance of known action that is a substance whose action on the living organism is known;
measuring a behavioral parameter of zebrafish not given the substance of known action in a bath having the zebrafish therein;
administering the substance of known action to the bath having the zebrafish therein and measuring the behavioral parameter of the zebrafish after the administration of the substance of known action in the bath having the zebrafish therein;
detecting change in the behavioral parameter of the zebrafish by the administration of the substance of known action;
subdividing data representing the change in the obtained behavioral parameter and converting the data into a large number of numerical data;
normalizing the large number of numerical data obtained based on the change in the behavioral parameter;
normalizing a large number of numerical data representing the change in expression levels of genes after the administration of the substance of known action; and
integrating and recording the large number of numerical data obtained based on the change in the behavioral parameter and the large number of numerical data representing change in expression levels of genes, both normalized.

The production of this library is described below.

The substance of known action to be used for the production of the library is selected in consideration of the action on the living organism focused in the production of the library, the predicted change in the behavioral parameters, and the like.

Preferred examples of the substance of known action to be used include active ingredients of pharmaceutical preparations. This is because as to active ingredients of pharmaceutical preparations, the relation between the administration of the active ingredient of the pharmaceutical preparation and the change in expression levels of genes often have been already known and therefore the action on the living organism and the change in expression levels of genes can be linked by referring to known documents. Moreover, they are preferred because the amount thereof to be used may be small.

Moreover, active ingredients of pharmaceutical preparations are preferred in that change in behavioral parameters of zebrafish would appear clearly since the action thereof on the living organism is strong.

The substance of known action to be used in the method for producing a library according to the present invention is preferably a substance having an already known relation between the administration of the substance of known action and change in expression levels of genes, the step of normalizing a large number of numerical data representing the change in expression levels of genes after the administration of the substance of known action preferably includes the steps of: obtaining a large number of numerical data representing change in expression levels of genes after the administration of the substance of known action; and normalizing the large number of numerical data, with reference to the relation already known.

If the relation between the administration of the substance of known action and change in expression levels of genes is already known, then, by measuring change in behavioral parameters of zebrafish when the substance of known action is administered, numerical data obtained by normalizing the large number of numerical data obtained based on change in the behavioral parameters of zebrafish and numerical data obtained by normalizing the large number of numerical data representing change in expression levels of genes can be integrated and stored as data in a library.

When the change in the behavioral parameters of zebrafish is measured when the substance of known action is administered, the behavioral parameters of zebrafish in the well to which the substance of known action is not administered are simultaneously measured as a control to be compared.

Since measuring the change in the behavioral parameters and the like of zebrafish when the substance of known action is administered provides a large number of numerical data based on change in the behavioral parameters and the like, the large number of numerical data are normalized.'

The normalization of the large number of numerical data obtained based on the change in the behavioral parameters is necessary for the comparison between experiments.

The normalization as used herein means to transform a large number of numerical data according to a certain rule to make them easy to be used, and refers to an operation of converting data so as to be mutually comparable probabilistically and statistically.

Examples of techniques for the normalization include methods using the Z score.

The Z score is calculated in the following formula and this value can be used for the cluster analysis.

$$Z \text{ score} = (\text{raw data value} - \text{control mean value})/\text{control standard deviation}$$

The methods for normalizing the large number of numerical data obtained based on the change in the behavioral parameters are not limited to the methods using the Z score, but other methods can be used.

As the substance of known action, a substance having central nervous system effect is preferably used and an active ingredient of a pharmaceutical preparation having central nervous system effect is preferably used.

Examples of the central nervous system effect include sedation, hypnosis, anesthetic action, excitation action, antidepressant action, depressive action, antianxiety, antihypnotic action, sleep action, and the like.

Examples of the active ingredient of a pharmaceutical preparation having central nervous system effect include fluphenazine dihydrochloride, diazepam, clonazepam C-IV, amitriptyline hydrochloride, haloperidol, chlorpromazine, suvorexant, imipramine, and the like.

Moreover, as the substance of known action, a substance having peripheral nervous system effect or muscular action is preferably used and an active ingredient of a pharmaceutical preparation having peripheral nervous system effect or muscular action is preferably used.

As for the active ingredient of any pharmaceutical preparation, the relation between the administration of the active ingredient of the pharmaceutical preparation and the change in expression levels of genes has been already known and it can be found by referring to GEO Datasets (NCBI) https://www.ncbi.nlm.nih.gov/gds/.

If the relation between the administration of the substance of known action and change in expression levels of genes is known, then, after citing array data from the database [GEO Datasets (NCBI)], the data in the array are normalized.

As a procedure of the normalization of data in the array, specifically, the procedure of (1) to (3) may be used and these can be performed using software in Gene Springs.

(1) Normalization to 75% percentile shift.

Based on the assumption that there is no change in overall expression quantity of genes and there is hardly any variation of percentile values between arrays, 75% of each sample is corrected to 0.

(2) Baseline correction

The ratio to the control sample is used.

(3) Extraction of Log2 FC (Fold Change) value in each GeneSymbol.

Common GeneSymbol among the arrays is extracted to get the Log2 FC value.

Subsequently, data among arrays are normalized. The normalization can be performed by methods using Z score like the normalization of the numerical data obtained based on the change in behavioral parameters.

The Z score is calculated in the following formula and this value can be used for the cluster analysis.

$$\text{Z score} = (\text{raw data value} - \text{control mean value})/\text{control standard deviation}$$

The methods for normalizing the data among arrays are not limited to the methods using the Z score, but other methods can be used.

Since the data obtained by normalizing the large number of numerical data obtained based on the change in the behavioral parameters and the like and the data obtained by normalizing the large number of numerical data representing the change in expression levels of genes are obtained for the substance of known action, these are integrated and recorded.

This integration can be performed, for example, on the basis of a statistical technique using the multivariate analysis, and the large number of numerical data obtained based on change in the behavioral parameters and the like and the large number of numerical data representing change in expression levels of genes, both normalized can be integrated and recorded by arraying on the same axis.

By this procedure, the data for the substance of known action are recorded in the library.

The precision of the library can be improved by carrying out a similar operation for other substances of known action to increase the number of substances of known action recorded in the library.

If the substance of known action for producing the library is a substance having an unknown relation between the administration of the substance of known action and change in expression levels of genes, then a large number of normalized numerical data representing change in expression levels of genes after the administration of the substance of known action need to be obtained for producing the library without depending on a database.

The step of obtaining the large number of numerical data representing change in expression levels of genes after the administration of the substance of known action may include, for example, the following steps of:

extracting mRNA from each of control zebrafish not given the substance of known action and zebrafish after the administration of the substance of known action, and measuring expression quantities of mRNAs to determine a sequence of mRNA whose expression quantity has been changed; and

identifying a gene whose expression level has changed correlationally by the administration of the substance of known action based on the sequence of the mRNA whose expression quantity has changed in a comparison between the control zebrafish and the zebrafish after the administration of the substance of known action, and

obtaining a large number of numerical data representing change in expression levels of genes.

Examples of the method for extracting mRNA from each zebrafish, measuring the expression quantities of mRNAs, and identifying the sequence of an mRNA whose expression level has changed include RNA Seq and DNA micro array, and the like, but the method may be another method.

When the change in the behavioral parameters of zebrafish after the administration of the substance of known action is measured, the behavioral parameters of control zebrafish in a well to which the substance of known action is not administered are simultaneously measured and mRNA is also extracted from the control zebrafish to measure the expression quantities of mRNAs and identify the sequence of an mRNA whose expression level has changed,

In this way, the expression quantities of the mRNA in the zebrafish after the administration of the substance of known action and the control zebrafish can be compared to identify a gene whose expression level is changed in correlation with the administration of the substance of known action.

Having the large number of numerical data representing change in expression levels of genes after the administration of the substance of known action obtained as described above as data in the array, the normalization of the data in the array is performed in the procedure (1) to (3) described above using software in Gene Springs.

Successively, data among arrays are normalized by a method using the Z score.

In this way, data obtained by normalizing a large number of numerical data representing change in expression levels of genes are obtained similarly to the case where the relation between the administration of the substance of known action and change in expression levels of genes is known.

Also for a substance having an unknown relation between the administration of the substance of known action and change in expression levels of genes, a large number of numerical data obtained based on change in behavioral parameters can be obtained and these large number of numerical data can be normalized.

As a technique for the normalization, the Z score can be used.

The methods for normalizing the data among arrays and the large number of numerical data obtained based on change in the behavioral parameters are not limited to the methods using the Z score, but other methods can be used.

Since the data obtained by normalizing the large number of numerical data obtained based on the change in the behavioral parameters and the like and the data obtained by normalizing the large number of numerical data representing the change in expression levels of genes are obtained for the substance of known action, these are integrated and recorded.

By such a method, a large number of numerical data obtained based on change in behavioral parameters of zebrafish after the administration of the substance of known action and a large number of numerical data representing change in expression levels of genes can be integrated and recorded in a library if the substance of known action is a substance having a known or unknown relation between the administration of the substance of known action and change in expression levels of genes.

In this way, a library for use in prediction of the action of a substance of interest on the living organism can be produced.

In the method for producing a library according to the present invention and the method for evaluation according to the present invention, stress may be given in zebrafish when the behavioral parameters of zebrafish are measured.

By giving stress, change in behavioral parameters in the presence of stress can be measured to measure the change in behavioral parameters found specifically when a substance of interest is given in the presence of stress. Moreover, a gene whose expression level is changed specifically in correlation with the administration of the substance of interest in the presence of stress can be identified.

Examples of the type of the stress include the repetition of light and dark periods, light period stimulation at a certain time, change in water temperature, stimulation with a chemical substance, infrared stimulation, ultraviolet stimulation, electrical stimulation, sound stimulation, vibration stimulation, and the like.

The action on the living organism to be evaluated by the method for evaluation according to the present invention is preferably central nervous system effect, peripheral nervous system effect, or muscular action. The method for evaluation according to the present invention is suitable for evaluating the central nervous system effect, the peripheral nervous system effect, or the muscular action.

The substance to be used as a substance of interest in the method for evaluation according to the present invention is not limited, but it is preferably a pharmaceutical preparation, an active ingredient of a pharmaceutical preparation, a food, or a component derived from a food, more preferably a food or a component derived from a food.

The food encompasses a food other than beverage, beverage, and food additives.

The component derived from a food means one obtained by isolating a substance included in the food.

As for substances already known as active ingredients of pharmaceutical preparations, their actions are considered to be already known, and therefore, the need to predict their actions on the living organism using the method for evaluation according to the present invention is low. However, among foods, there are many foods whose actions on the living organism are not known and it is useful to predict their actions on the living organism.

Examples of the substance that can be used as a substance of interest and that is a component derived from a food include epigallocatechin gallate, 4-amino-n-butyric-acid (GABA), L-theanine, 8beta-(4'-hydroxytigloyloxy)costunolide (8beta), and the like.

Hereinafter, an example where for substances having central nervous system effect, the measurement of behavioral parameters of zebrafish and identification of genes whose expression levels are changed in correlation with the administration of the substances are performed is described as an example of a specific embodiment of the present invention.

[Preparation of water bath]

$0.3 \times$ Danieau's solution (19.3 mM NaCl, 0.23 mM KCl, 0.23 mM MgSO$_4$, 0.2 mM Ca(NO$_3$)$_2$, 1.7 mM HEPES, pH 7.2) is used as a water bath.

Hereinafter, the water bath in the description of an example of this embodiment refers to this water bath.

[Preparation of zebrafish]

Zebrafish is prepared by growing it under the following conditions to 6 dpf.

Zebrafish: AB Line 6 dpf
Water bath temperature: 28°C
The repetition of light and dark periods during the growth: Light period 14 hours (from 7:00 to 21:00) - Dark period 10 hours (from 21:00 to 7:00)

[Preparation of reagents for production of library]

The following substances (active ingredients of pharmaceutical preparations) are used as substances of known action for the production of a library.

Diazepam (indicated as Diazepam in the figures)
Haloperidol (indicated as Haloperidol in the figures)

For each substance of known action, the reagent in which the substance was dissolved in the water bath and adjusted at a concentration of 10 μM was prepared.
These substances of known action are both substances having central nervous system effect and the relations between the administration of the substances and changes in expression levels of genes are already known.

[Measurement procedure of behavioral parameters]

(1) Add 1 ml of the water bath to each well of a 48-well plate. Transfer one zebrafish (6 dpf) per well thereto and adjust the temperature to 28°C and maintain the temperature for from 30 minutes to 1 hour.
(2) Remove the liquid in the 48-well plate with a pipette and then add 1 ml/well of any of the reagents.
(3) Set the 48-well plate in Danio Vision (manufactured by Noldus Information Technology) and record the behavior. The repetition of light and dark periods with cycled of a light period for 3 hours (period L1) - a dark period for 10 hours (period D) - a light period for 11 hours (period L2) is performed and the behavior of zebrafish is recorded for 24 hours.
(4) Perform the behavioral analysis by using a software (Ethovision, manufactured by Noldus Information Technology). Mobility states are classified into 3 stages of high speed (mobility states equal to or higher than 65 and equal to or lower than 95), moderate speed (mobility states equal to or higher than 35 and lower than 65), low speed (mobility states equal to or higher than 5 and lower than 35).

For each class of mobility states, the indicators DMF, IZF, DTZ, and TA are measured. The data of DMF, IZF, DTZ, and TA for each class of mobility states are divided for the period L1, the period D, and the period L2, which are the periods in the repetition of light and dark periods.

[Production of library]

(1) In a 48-well plate, prepare wells in which 1 ml of each reagent prepared in [Preparation of reagents for production of library] and as a control wells in which 1 ml of a control water bath made by adding DMSO to the water bath so that the concentration of DMSO is the same as that of DMSO contained in each reagent, transfer 6 dpf zebrafish prepared in [Preparation of zebrafish] to the wells, and measure the behavioral parameters according to [Measurement procedure of behavioral parameters].
If the reagent does not contain DMSO, then it is not necessary to add DMSO to the control reagent.
(2) Store the measured behavioral parameters as raw data of the following forms.

- Type of reagent (any of the reagents prepared in [Preparation of reagents for production of library] or control)
- Mobility state (95 to 65 (high speed), 65 to 35 (moderate speed), or 35 to 05 (low speed))
- Repetition of light and dark periods (L1, D, or L2)
- Indicator (DMF, IZF, DTZ, or TA)

If there are 3 mobility states, 3 periods in the repetition of light and dark periods, and 4 indicators, then the number of raw data is $3 \times 3 \times 4 = 36$. But a higher number of raw data can actually be obtained by changing conditions of the repetition of light and dark periods and/or conditions of other stimulation, and the like.

(3) Detect the difference between the stored indicators of the behavioral parameters at the time of use of the substance of known action and the behavioral parameters of the control. The difference is the change in the behavioral parameters.

(4) Normalize the change in the behavioral parameters using the Z score. The method for the normalization with the Z score is as described above.

(5) Subsequently, link the administration of the substance of known action and the change in expression levels of genes.

This can be done by citing array data of Diazepam (GSE23157) and Haloperidol (GSE89780) from a public DB (Geo Datasets).

Based on these array data, the normalization of the data in the arrays is performed in the procedure described above using software in Gene Springs and data among arrays are normalized by a method using the Z score.

(6) Integrate and record, in a library, data obtained by normalizing the large number of numerical data obtained based on the change in the behavioral parameters and data obtained by normalizing the large number of numerical data representing change in expression levels of genes obtained for the substances of known action Diazepam and Haloperidol as seen above.

[Evaluation of action of substance of interest on living organism]

An example involving use of epigallocatechin gallate (hereinafter, referred to as EGCG in the description and the figures) as a substance of interest is described below.

(1) Prepare a reagent made by dissolving EGCG in the water bath and adjusting the concentration thereof to 0.003 mg/mL.

In a 48-well plate, prepare wells in which 1 ml of the reagent made by dissolving EGCG in the water bath is transferred and wells in which only 1 ml of the water bath is transferred as a control, transfer 6 dpf zebrafish prepared in [Preparation of zebrafish] to the wells, and measure the behavioral parameters according to [Measurement procedure of behavioral parameters].

(2) Store the measured behavioral parameters as raw data in the same procedure as [Production of library], detect the behavioral parameters, and normalize the change in the behavioral parameters using the Z score.

(3) Link the administration of EGCG and the change in expression levels of genes using microarray experiments.

The microarray experiments can be performed using Zebrafish oligo-DNA micro array Ver.3.0 manufactured by Agilent Technologies, Inc. according to protocol.

Based on array data obtained by this microarray experiment, the normalization of the data in the arrays is performed in the procedure described above using software in Gene Springs and data among arrays are normalized by a method using the Z score.

(4) In this way, data obtained by normalizing the large number of numerical data obtained based on the change in the behavioral parameters are obtained for EGCG. Moreover, data obtained by normalizing a large number of numerical data representing change in expression levels of genes when EGCG is administered are obtained.

[Analysis by statistical matching]

Processing of statistically matching and analyzing the numerical data for Diazepam and Haloperidol recorded in the library and the numerical data with the administration of EGCG was conducted by the cluster analysis as follows.

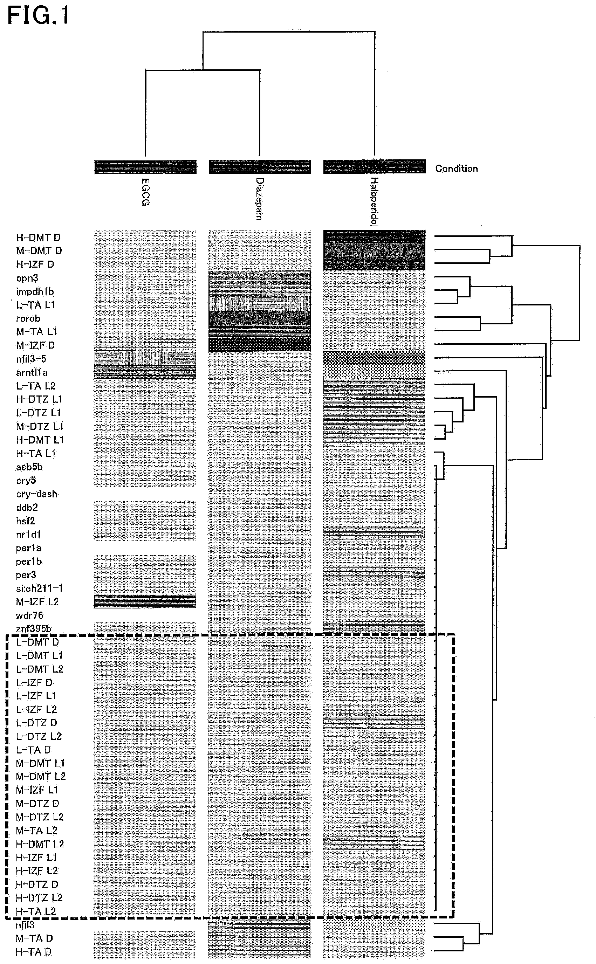

FIGs. 1 to 3 illustrate the results of the cluster analyses.

FIG. 1 illustrates the result of the cluster analysis with the Euclid distance.

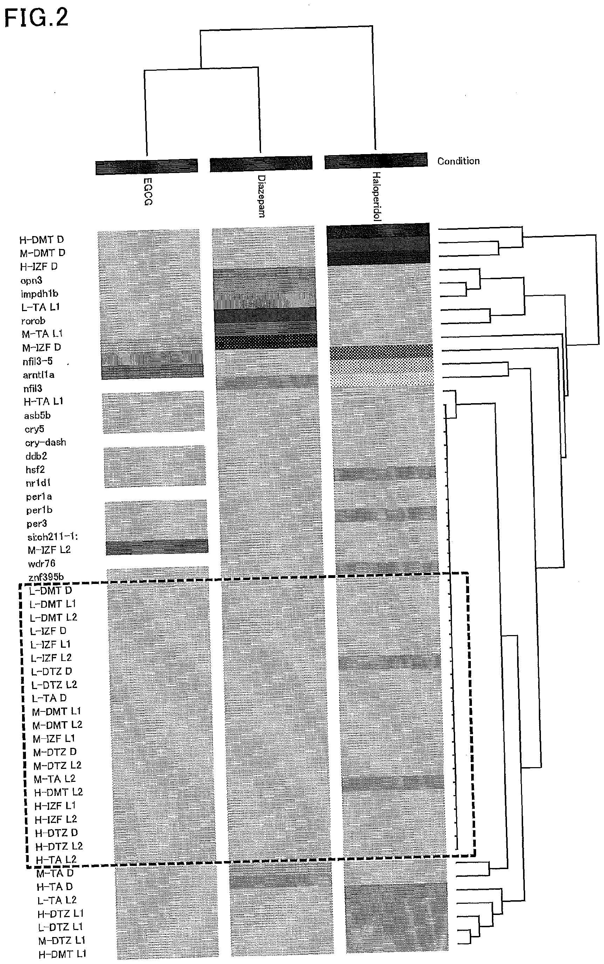

FIG. 2 illustrates the result of the cluster analysis with the Manhattan distance.

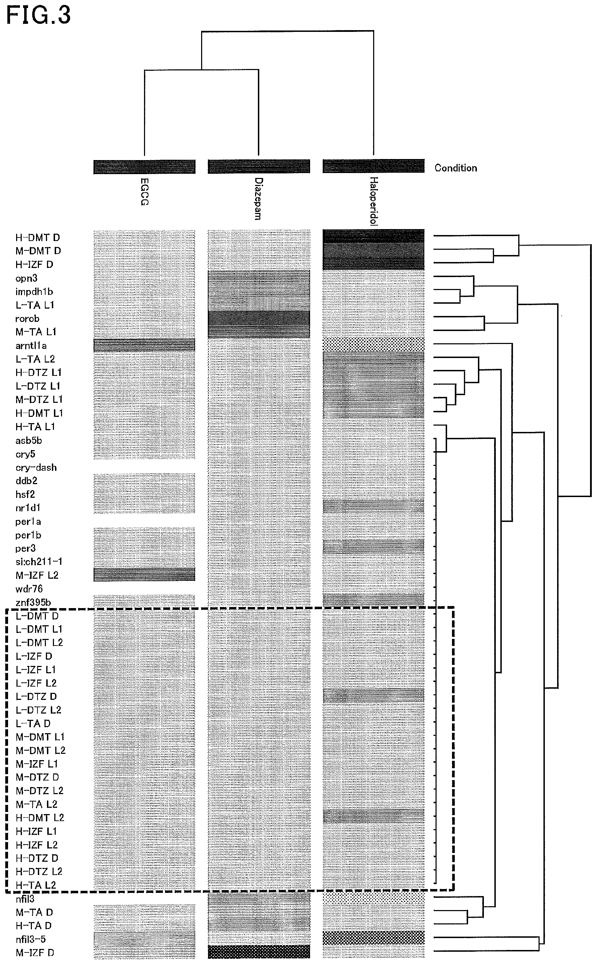

FIG. 3 illustrates the result of the cluster analysis with the maximum distance.

In each figure, the notions starting with L-, M-, and H- (36 in total) indicate data of change in the behavioral parameters. The remaining lower case alphabet notions (18 in total) indicate data of change in expression levels of genes (Gene Symbol).

The Gene Symbol shown in each figure is Gene Symbol for which the expression level of the gene is significantly changed extracted from a large number of numerical data representing change in expression levels of genes by a technique using software in Gene Springs as described herein above.

In these figures, the data of the change in the behavioral parameters and the data of the change in expression levels of genes are shown together.

The pattern and darkness of each hatching reflect the Z score after the normalization of the data of the change in the behavioral parameters and the data of the change in gene expression. The hatching with dots indicates the amount of change of the Z score being the - side and the hatching with filling indicates the amount of change of the Z score being the + side and the darker color indicates the larger absolute value of the amount of change of the Z score.

First, the behavioral parameters in the region surrounded with the squares indicated with the dotted lines in FIGs 1 to 3 are found to be similar between EGCG and Diazepam from the result of the cluster analysis of the behavioral parameters. This suggests that EGCG is closely related to Diazepam. As seen above, identification of closely related pharmaceutical preparations makes it possible to estimate the action and the phenotype of the substance of interest from the drug information. The drug information can be searched in databases such as KEGG MEDICUS drug information or iyakuSearch.

Since Diazepam is known to have sedative action, it is estimated that EGCG has sedative action too.

Moreover, if a large number of numerical data representing change in expression levels of genes expressed by the administration of a substance of interest can be obtained, then novel action or mechanism of the substance of interest can be estimated in more detail by using, statistically matching, and analyzing a large number of numerical data representing change in expression levels of genes expressed by the administration of the substance of interest.

Specifically, it is possible by statistically matching and analyzing the large number of numerical data obtained based on change in behavioral parameters of zebrafish by the administration of the substance of interest and the large number of numerical data representing the change in expression levels of genes after the administration of the substance of interest, both normalized; and the large number of numerical data obtained based on change in the behavioral parameters of zebrafish and a large number of numerical data representing change in expression levels of genes integrated and recorded in the library.

Examples of the technique to obtain a large number of numerical data representing change in expression levels of genes expressed by the administration of the substance of interest include the step of microarray experiment and normalization illustrated in the item (3) in [Evaluation of action of substance of interest on living organism] above.

An example in which novel action and mechanism of the substance of interest, when EGCG is the substance of interest, are estimated using data obtained in this step of microarray experiment and normalization is as follows.

In the result of cluster analysis including data of change in expression levels of genes, the action and mechanism of EGCG can be estimated based on the Gene Ontrogy (GO) information of the gene expression data by paying attention on data having high correlation between EGCG and Diazepam (data clustered in close relation). GO can be searched in a database such as AmiGO 2.

Examples of the gene expression data (Gene Symbol) that change with correlation between EGCG and Diazepam based on the result of cluster analysis include perlb. This gene forms a closely related cluster with behavioral analysis data such as L-DMT D, L-DMT L1, and L-DMT L2. Gene Ontrogy data of perlb are GO: 0009266 | GO: 0071542 | GO: 0042752 | GO: 0009416 and these indicate action on the regulation of the circadian rhythm and the differentiation of dopaminergic neuron. As seen above, a possibility that novel action and mechanism of a substance of interest can be estimated by making use of the GO analysis and the pathway analysis based on the result of cluster analysis is indicated.

Hereinafter, an example where for substances having central nervous system effect (in particular, sleep action), the measurement of behavioral parameters of zebrafish and identification of genes whose expression levels are changed in correlation with the administration of the substances are performed is described as another example of a specific embodiment of the present invention.

[Preparation of water bath]

$0.3 \times$ Danieau's solution (19.3 mM NaCl, 0.23 mM KCl, 0.23 mM $MgSO_4$, 0.2 mM $Ca(NO_3)_2$, 1.7 mM HEPES, pH 7.2) is used as a water bath, and a 0.1% DMSO-containing water bath is used.

Hereinafter, the water bath in the description of an example of this embodiment refers to this water bath.

[Preparation of zebrafish]

Zebrafish is prepared by growing it under the following conditions to 8 dpf.

Zebrafish: MieKomachi 002 Line 8 dpf
Water bath temperature: 28°C

The repetition of light and dark periods during the growth: Light period 14 hours (from 5:00 to 19:00) - Dark period 10 hours (from 19:00 to 5:00)

[Preparation of reagents for production of library]

The following substances (active ingredients of pharmaceutical preparations) are used as substances of known action for the production of a library.

Suvorexant (indicated as Suvorexant in the figures)
Imipramine (indicated as Imipramine in the figures)

For each substance of known action, the reagent in which the substance was dissolved in the water bath and adjusted at a concentration of 1 $\mu$M was prepared.

These substances of known action are both substances having central nervous system effect and the relations between the administration of the substances and changes in expression levels of genes are already known.

[Measurement procedure of behavioral parameters]

(1) Add 1 ml of the water bath to each well of a 48-well plate. Transfer one zebrafish (8 dpf) per well thereto and adjust the temperature to 28°C and maintain the temperature for from 30 minutes to 1 hour.

(2) Remove the liquid in the 48-well plate with a pipette and then add 1 ml/well of any of the reagents.

(3) Set the 48-well plate in Danio Vision (manufactured by Noldus Information Technology) and record the behavior. The repetition of light and dark periods with cycled of a light period for 3 hours (period L1) - a dark period for 10 hours (period D) - a light period for 11 hours (period L2) is performed and the behavior of zebrafish is recorded for 24 hours.

(4) Perform the behavioral analysis by using a software (Ethovision, manufactured by Noldus Information Technology). Mobility states are classified into 3 stages of high speed (mobility states equal to or higher than 65 and equal to or lower than 95), moderate speed (mobility states equal to or higher than 35 and lower than 65), low speed (mobility states equal to or higher than 5 and lower than 35).

For each class of mobility states, the indicators DMF, IZF, DTZ, and TA are measured. The data of DMF, IZF, DTZ, and TA for each class of mobility states are divided for the period L1, the period D, and the period L2, which are the periods in the repetition of light and dark periods.

[Production of library]

(1) In a 48-well plate, prepare wells in which each reagent prepared in [Preparation of reagents for production of library] and as a control wells in which a control water bath made by adding DMSO to the water bath so that the concentration of DMSO is the same as that of DMSO contained in each reagent, transfer 8 dpf zebrafish prepared in [Preparation of zebrafish] to the wells, and measure the behavioral parameters according to [Measurement procedure of behavioral parameters].

(2) Store the measured behavioral parameters as raw data of the following forms.

- Type of reagent (any of the reagents prepared in [Preparation of reagents for production of library] or control)
- Mobility state (95 to 65 (high speed), 65 to 35 (moderate speed), or 35 to 05 (low speed))
- Repetition of light and dark periods (L1, D, or L2)
- Indicator (DMF, IZF, DTZ, or TA)

If there are 3 mobility states, 3 periods in the repetition of light and dark periods, and 4 indicators, then the number of raw data is $3 \times 3 \times 4 = 36$. But a higher number of raw data can actually be obtained by changing conditions of the repetition of light and dark periods and/or conditions of other stimulation, and the like.

(3) Detect the difference between the stored indicators of the behavioral parameters at the time of use of the substance of known action and the behavioral parameters of the control. The difference is the change in the behavioral parameters.

(4) Normalize the change in the behavioral parameters using the Z score. The method for the normalization with the Z score is as described above.

(5) Subsequently, link the administration of the substance of known action and the change in expression levels of genes using microarray experiments.

The microarray experiments can be performed using Zebrafish oligo-DNA micro array Ver.3.0 manufactured by Agilent Technologies, Inc. according to protocol.

Based on array data obtained by this microarray experiment, the normalization of the data in the arrays is performed in the procedure described above using software in Gene Springs.

(6) Integrate and record, in a library, data obtained by normalizing the large number of numerical data obtained based on the change in the behavioral parameters and data obtained by normalizing the large number of numerical data representing change in expression levels of genes obtained for the substances of known action Suvorexant and Imipramine as seen above.

[Evaluation of action of substance of interest on living organism]

Hereinafter, an example in which 8beta-(4'-hydroxytigloyloxy)costunolide (hereinafter, referred to as 8beta in the specification and the figures), which can be used as a food material, is used as a substance of interest is described.

(1) Prepare a reagent made by dissolving 8beta in the water bath and adjusting the concentration thereof to 10 μM. In a 48-well plate, prepare wells in which 1 ml of the reagent made by dissolving 8beta in the water bath is transferred and wells in which only 1 ml of the water bath is transferred as a control, transfer 8 dpf zebrafish prepared in [Preparation of zebrafish] to the wells, and measure the behavioral parameters according to [Measurement procedure of behavioral parameters].

(2) Store the measured behavioral parameters as raw data in the same procedure as [Production of library], detect the behavioral parameters, and normalize the change in the behavioral parameters using the Z score.

(3) Link the administration of 8beta and the change in expression levels of genes using microarray experiments.

The microarray experiments can be performed using Zebrafish oligo-DNA micro array Ver.3.0 manufactured by Agilent Technologies, Inc. according to protocol.

Based on array data obtained by this microarray experiment, the normalization of the data in the arrays is performed in the procedure described above using software in Gene Springs and data among arrays are normalized by a method using the Z score.

(4) In this way, data obtained by normalizing the large number of numerical data obtained based on the change in the behavioral parameters are obtained for 8beta. Moreover, data obtained by normalizing a large number of numerical data representing change in expression levels of genes when 8beta is administered are obtained.

[Analysis by statistical matching]

Processing of statistically matching and analyzing the numerical data for Suvorexant and Imipramine recorded in the library and the numerical data with the administration of 8beta was conducted by the cluster analysis as follows.

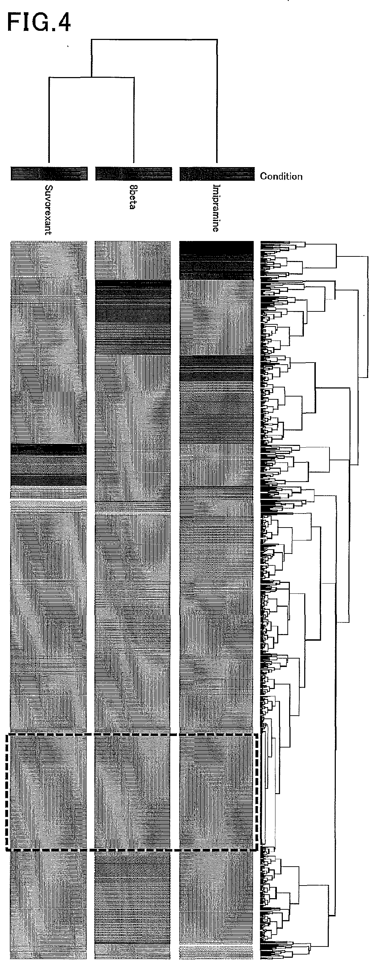

FIG. 4 illustrates the result of the cluster analysis with the Euclid distance.

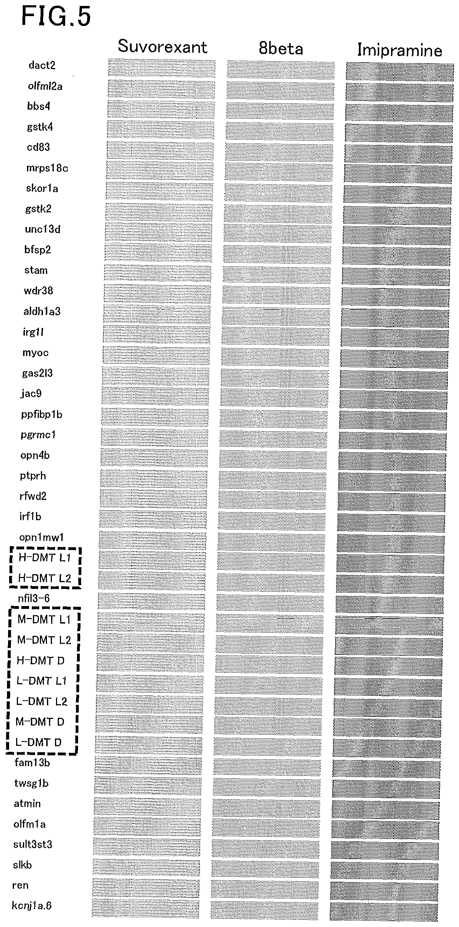

FIG. 5 is an enlarged view of the region surrounded with a dotted line in FIG. 4.

In FIG. 5, the notions starting with L-, M-, and H-indicate data of change in the behavioral parameters. The remaining lower case alphabet notions indicate data of change in expression levels of genes (Gene Symbol).

The Gene Symbol shown in each figure is Gene Symbol for which the expression level of the gene is significantly changed extracted from a large number of numerical data representing change in expression levels of genes by a technique using software in Gene Springs as described herein above.

In these figures, the data of the change in the behavioral parameters and the data of the change in expression levels of genes are shown together.

The pattern and darkness of each hatching reflect the Z score after the normalization of the data of the change in the behavioral parameters and the data of the change in gene expression.

First, the behavioral parameters in the region indicated with the dotted lines in FIG. 5 are found to be similar between 8beta and Suvorexant from the result of the cluster analysis of the behavioral parameters. This suggests that 8beta is closely related to Suvorexant. As seen above, identification of closely related pharmaceutical preparations makes it possible to estimate the action and the phenotype of the substance of interest from the drug information. The drug information can be searched in databases such as KEGG MEDICUS drug information or iyakuSearch.

Since Suvorexant is known to have sleep action, it is estimated that 8beta has sleep action too.

Moreover, if a large number of numerical data representing change in expression levels of genes expressed by the administration of a substance of interest can be obtained, then novel action or mechanism of the substance of interest can be estimated in more detail by using, statistically matching, and analyzing a large number of numerical data representing change in expression levels of genes expressed by the administration of the substance of interest.

Specifically, it is possible by statistically matching and analyzing the large number of numerical data obtained based on change in behavioral parameters of zebrafish by the administration of the substance of interest and the large number of numerical data representing the change in expression levels of genes after the administration of the substance of interest, both normalized; and the large number of numerical data obtained based on change in the behavioral parameters of zebrafish and a large number of numerical data representing change in expression levels of genes integrated and recorded in the library.

Examples of the technique to obtain a large number of numerical data representing change in expression levels of genes expressed by the administration of the substance of interest include the step of microarray experiment and normalization illustrated in the item (3) in [Evaluation of action of substance of interest on living organism] above.

An example in which novel action and mechanism of the substance of interest, when 8beta is the substance of interest, are estimated using data obtained in this step of microarray experiment and normalization is as follows.

In the result of cluster analysis including data of change in expression levels of genes, the action and mechanism of 8beta can be estimated based on the Gene Ontrogy (GO) information of the gene expression data by paying attention on data having high correlation between 8beta and Suvorexant (data clustered in close relation). GO can be searched in a database such as AmiGO 2.

Examples of the gene expression data (Gene Symbol) that change with correlation between 8beta and Suvorexant based on the result of cluster analysis include nfil3-6. This gene forms a closely related cluster with behavioral analysis data such as H-DMT D, H-DMT L1, H-DMT L2, M-DMT D, M-DMT L1, M-DMT L2, L-DMT D, L-DMT L1, and L-DMT L2. Gene Ontrogy data of nfil3-6 are GO: 0043565 | GO: 0001078 | GO: 0006355 | GO: 0003700 | GO: 0005634 | GO: 0032922 and these are involved in the regulation of the circadian rhythm. As seen above, a possibility that novel action and mechanism of a substance of interest can be estimated by making use of the GO analysis and the pathway analysis based on the result of cluster analysis is indicated.

The animal experiments using zebrafish were conducted in accordance with Law of Humane Treatment and Management of Animals and related regulations.

INDUSTRIAL AVAILABILITY

The present invention provides a method for exhaustively linking, with the administration of a substance of interest, and evaluating the change in expression levels of genes and the action of the substance of interest on the living organism using zebrafish. Moreover, a method for producing a library for use in prediction of the action of a substance of interest on the living organism is provided.

When the method for evaluation according to the present invention is used, exhaustive screening of foods and the like for the action on the living organism can be performed by a method that is low cost and easy in comparison with those in which rodents are used.

**Claims**

1. A method for producing a library for use in prediction of the action of a substance of interest on the living organism, the library having a large number of numerical data obtained based on change in a behavioral parameter of zebrafish and a large number of numerical data representing change in expression levels of genes integrated and recorded therein, the method comprising the steps of:

    preparing a substance of known action that is a substance whose action on the living organism is known;
    measuring a behavioral parameter of zebrafish not given the substance of known action in a bath having the zebrafish therein;
    administering the substance of known action to the bath having the zebrafish therein and measuring the behavioral parameter of the zebrafish after the administration of the substance of known action in the bath having the zebrafish therein;
    detecting change in the behavioral parameter of the zebrafish by the administration of the substance of known action;
    subdividing data representing the change in the obtained behavioral parameter and converting the data into a large number of numerical data;
    normalizing the large number of numerical data obtained based on the change in the behavioral parameter;
    normalizing a large number of numerical data representing the change in expression levels of genes after the administration of the substance of known action; and
    integrating and recording the large number of numerical data obtained based on the change in the behavioral parameter and the large number of numerical data representing change in expression levels of genes, both normalized.

2. The method for producing a library according to claim 1,
wherein the substance of known action is an active ingredient of a pharmaceutical preparation.

3. The method for producing a library according to claim 1 or 2,
wherein the substance of known action is a substance having an already known relation between the administration of the substance of known action and change in expression levels of genes, and
the step of normalizing a large number of numerical data representing the change in expression levels of genes after the administration of the substance of known action comprises the steps of:

obtaining a large number of numerical data representing change in expression levels of genes after the administration of the substance of known action; and
normalizing the large number of numerical data, with reference to the relation already known.

4. The method for producing a library according. to claim 1 or 2,
wherein the substance of known action is a substance having an unknown relation between the administration of the substance of known action and change in expression levels of genes, and
the step of normalizing a large number of numerical data representing change in expression levels of genes after the administration of the substance of known action comprises the steps of:

extracting mRNA from each of control zebrafish not given the substance of known action and zebrafish after the administration of the substance of known action, and measuring expression quantities of mRNAs to determine a sequence of mRNA whose expression quantity has been changed;
identifying a gene whose expression level has changed correlationally by the administration of the substance of known action based on the sequence of the mRNA whose expression quantity has changed in a comparison between the control zebrafish and the zebrafish after the administration of the substance of known action, and obtaining a large number of numerical data representing change in expression levels of genes after the administration of the substance of known action; and
normalizing the large number of numerical data.

5. The method for producing a library according to any of claims 1 to 4,
wherein the substance of known action is a substance having central nervous system effect.

6. The method for producing a library according to any of claims 1 to 5,
wherein the behavioral parameter is at least one behavioral parameter selected from the group consisting of the mobility state, the distance moved total, the in zone frequency, the distance to zone, and the turn angle of zebrafish.

7. A method for evaluating the action of a substance of interest on the living organism, comprising the steps of:

preparing a library having a large number of numerical data obtained based on change in a behavioral parameter of zebrafish and a large number of numerical data representing change in expression levels of genes, both normalized, integrated, and recorded therein;
measuring a behavioral parameter of zebrafish not given a substance of interest in a bath having the zebrafish therein;
administering the substance of interest to the bath having the zebrafish therein and measuring the behavioral parameter of the zebrafish after the administration of the substance of interest in the bath having the zebrafish therein;
normalizing a large number of numerical data obtained based on change in the behavioral parameter of zebrafish by the administration of the substance of interest;
identifying a gene whose expression level is changed in correlation with the administration of the substance of interest by statistically matching and analyzing the large number of numerical data obtained based on change in the behavioral parameter of zebrafish by the administration of the substance of interest and the large number of numerical data obtained based on change in the behavioral parameter of zebrafish and the large number of numerical data representing change in expression levels of genes integrated and recorded in the library, both normalized;
thereby predicting action of the substance of interest on the living organism based on function of the identified gene.

8. The method for evaluation according to claim 7,
wherein the behavioral parameter is at least one behavioral parameter selected from the group consisting of the mobility state, the distance moved total, the in zone frequency, the distance to zone, and the turn angle of zebrafish.

9. The method for producing a library according to any of claims 1 to 6 or method for evaluation according to claim 7 or 8, wherein the substance of interest is a food or a component derived from a food.

10. The method for evaluation according to any of claims 7 to 9, further comprising the steps of:

extracting mRNA from each of control zebrafish not given the substance of interest and zebrafish after the administration of the substance of interest, and measuring expression quantities of mRNAs to determine a sequence of mRNA whose expression quantity has been changed;
identifying a gene whose expression level has changed correlationally by the administration of the substance of interest based on the sequence of the mRNA whose expression quantity has changed in a comparison between the control zebrafish and the zebrafish after the administration of the substance of interest, and obtaining a large number of numerical data representing change in expression levels of genes after the administration of the substance of interest; and normalizing a large number of numerical data representing the change in expression levels of genes after the administration of the substance of interest; thereby obtaining the large number of numerical data representing the change in expression levels of genes after the administration of the substance of interest; and
estimating novel action or mechanism of the substance of interest by statistically matching and analyzing the large number of numerical data obtained based on change in the behavioral parameter of zebrafish by the administration of the substance of interest and the large number of numerical data representing the change in expression levels of genes after the administration of the substance of interest, both normalized, and the large number of numerical data obtained based on change in the behavioral parameter of zebrafish and a large number of numerical data representing change in expression level of a gene integrated and recorded in the library.

# FIG.1

# FIG.2

EP 3 733 873 A1

# FIG.3

# FIG.4

# FIG.5

## INTERNATIONAL SEARCH REPORT

| International application No. |
|---|
| PCT/JP2018/038789 |

**A. CLASSIFICATION OF SUBJECT MATTER**
Int.Cl. C12Q1/6851(2018.01)i, C40B50/06(2006.01)i, G01N33/02(2006.01)i, G01N33/15(2006.01)i, G01N33/50(2006.01)i

According to International Patent Classification (IPC) or to both national classification and IPC

**B. FIELDS SEARCHED**

Minimum documentation searched (classification system followed by classification symbols)
Int.Cl. C12Q1/6851, C40B50/06, G01N33/02, G01N33/15, G01N33/50

Documentation searched other than minimum documentation to the extent that such documents are included in the fields searched
Published examined utility model applications of Japan      1922-1996
Published unexamined utility model applications of Japan    1971-2019
Registered utility model specifications of Japan            1996-2019
Published registered utility model applications of Japan    1994-2019

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN), PubMed

**C. DOCUMENTS CONSIDERED TO BE RELEVANT**

| Category* | Citation of document, with indication, where appropriate, of the relevant passages | Relevant to claim No. |
|---|---|---|
| A | 村上宗一郎他, 新規ゼブラフィッシュ行動解析を用いたニコチン代替療法の発達神経毒性評価, 日本分子生物学会年会プログラム・要旨集, 2013, vol. 36, p. 3P-0729, (MURAKAMI, Soichiro et al.), non-official translation (Evaluation of developmental neurotoxicity of nicotine replacement therapy that be used for new zebrafish behavioral analysis, Program and Proceedings of the Molecular Biology Society of Japan) | 1-10 |

☒ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 10.01.2019 | 22.01.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2018/038789

C (Continuation).   DOCUMENTS CONSIDERED TO BE RELEVANT

| Category* | Citation of document, with indication, where appropriate, of the relevant passages | Relevant to claim No. |
|---|---|---|
| A | 芦川芳史他，新規ゼブラフィッシュ行動解析を用いた抗甲状腺薬の発達神経毒性評価，日本分子生物学会年会プログラム・要旨集，2013, vol. 36, p. 3P-0728, (ASHIKAWA, Yoshifumi et al.), non-official translation (Evaluation of developmental neurotoxicity of antithyroid drug that be used for new zebrafish behavioral analysis, Program and Proceedings of the Molecular Biology Society of Japan) | 1-10 |
| A | JP 2008-193912 A (MIE UNIV) 28 August 2008, entire text (Family: none) | 1-10 |
| A | JP 2011-55755 A (MIE UNIV) 24 March 2011, entire text (Family: none) | 1-10 |
| A | JOHNSON, A. and HAMILTON, T. J., Modafinil decreases anxiety-like behaviour in zebrafish, PeerJ, 2017, 5, e2994 | 1-10 |
| A | JP 2004-89027 A (JAPAN SCIENCE AND TECH AGENCY) 25 March 2004, entire text & WO 2004/021282 A1 | 1-10 |
| A | MAXIMINO, C. et al., Measuring anxiety in zebrafish: A critical review, Behav. Brain Res., 2010, vol. 214, pp. 157-171 | 1-10 |
| A | KILY, L. J. M., Gene expression changes in a zebrafish model of drug dependency suggest conservation of neuro-adaptation pathways, J. Exp. Biol., 2008, vol. 211, pp. 1623-1634 | 1-10 |
| A | 西村有平，神経疾患治療薬の開発におけるゼブラフィッシュの有用性，日薬理誌，2017, vol. 150, pp. 88-91, (NISHIMURA, Yuhei, Folia Pharmacologica Japonica), non-official translation (Utility of zebrafish in the development of nerve disease therapeutic drugs) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PORSOLT et al.** *Nature,* 1977, vol. 266, 730-732

- **JOHNSON A ; HAMILTON TJ.** Modafinil decreases anxiety-like behaviour in zebrafish. *PeerJ,* 2017, vol. 5, e2994